# EUROPÄISCHE PATENTSCHRIFT

(11) **EP 3 990 179 B1**
(45) Veröffentlichungstag und Bekanntmachung des Hinweises auf die Patenterteilung: **22.11.2023**
(21) Anmeldenummer: 20734129.8
(22) Anmeldetag: 16.06.2020
(51) Int. Cl.: B01L 3/00, A61B 5/15, A61B 5/154

(54) **VERSCHLUSSKAPPE ZUM VERSCHLIESSEN EINES PROBENRÖHRCHENS ZUR AUFNAHME EINER FLÜSSIGKEIT**
SEALING CAP FOR SEALING A SAMPLE TUBE FOR RECEIVING A LIQUID
BOUCHON DE FERMETURE POUR FERMER UN TUBE POUR PRÉLÈVEMENT DESTINÉ À RECEVOIR UN LIQUIDE

(30) Priorität: 26.06.2019 DE 102019117240
(43) Veröffentlichungstag der Anmeldung: 04.05.2022
(73) Patentinhaber: Sarstedt AG & Co. KG, 51588 Nümbrecht (DE)
(72) Erfinder: WEINSTOCK, Mark, 57612 Helmenzen (DE)
(74) Vertreter: Grosse, Wolf-Dietrich Rüdiger
(86) Internationale Anmeldenummer: PCT/EP2020/066578
(87) Internationale Veröffentlichungsnummer: WO 2020/260066

(56) Entgegenhaltungen:
- WO-A1-2015/106191
- WO-A2-2008/067215
- US-A- 4 150 089

## Beschreibung

### Gebiet

Die Erfindung betrifft eine Verschlusskappe zum Verschließen eines Probenröhrchens zur Aufnahme einer Flüssigkeit, insbesondere einer menschlichen oder tierischen Flüssigkeit, weiter insbesondere Blut. Weiterhin wird ein Blutentnahmeröhrchen, u.a. aufweisend die erfindungsgemäße Verschlusskappe und ein Verfahren zur Handhabung des Blutentnahmeröhrchens beansprucht.

### Stand der Technik

Hauptanforderung an ein Blutentnahmeröhrchen, bestehend aus einem Probenröhrchen und einer Verschlusskappe, zur Entnahmen von Proben von Flüssigkeiten aus einem Körper ist es, das es dicht schließt und eine Kontamination der Proben verhindert.

Aus der internationalen Patentanmeldung WO2017/162 574 A1 sowie der japanischen Patentanmeldung JP2005 287 955 sind Verschlusskappen für Probenröhrchen grundsätzlich bekannt. Die Verschlusskappen weisen auf ihrer zum Probenröhrchen zeigenden Seite ein Rückschlagventil auf. Die gegenüberliegende Seite wird mit einer Membran verschlossen, die mit einer Kanüle durchstochen werden kann. Dazwischen ist ein Hohlraum ausgebildet. Mit Hilfe der Kanüle kann eine Flüssigkeit, z.B. aus einem menschlichen oder tierischen Körper, entnommen werden. Die Flüssigkeit aus dem Körper strömt durch die Kanüle in den Hohlraum in der Verschlusskappe und anschließend durch das geöffnete Rückschlagventil in das Probenröhrchen. Wird die Kanüle aus der Verschlusskappe gezogen, verschließt sich die Membran selbsttätig. Durch eine entsprechende Ausgestaltung des Rückschlagventils wird gewährleistet, dass das Probenröhrchen sicher gegen einen Austritt der Flüssigkeit verschlossen ist. Es werden verschiedene Ausführungsformen für Rückschlagventile offenbart, die sich jeweils unter Einwirkung einer Zentrifugalkraft in axialer Richtung der Probenröhrchen öffnen.

Zur Vorbereitung der Flüssigkeit aus dem Körper, insbesondere Blut für eine spätere Analyse, ist es üblich und erforderlich, diese in ihre leichten und schweren Bestandteile aufzutrennen. Dies erfolgt typischerweise durch eine Zentrifugation der Flüssigkeit in dem Blutentnahmeröhrchen.

Vor einer Probenahme, d. h. vor der Befüllung des Blutentnahmeröhrchens mit der Flüssigkeit, wird in der Regel herstellerseitig ein Gerinnungshemmer oder ein Gerinnungsbeschleuniger, welche beide nachfolgend auch als Präparation bezeichnet werden, in das Probenröhrchen eingebracht. In dem Blutentnahmeröhrchen mischt sich die Präparation mit der einströmenden Flüssigkeit. Die Präparation stellt sicher, dass die Blutbestandteile für die spätere Analyse in einem gewünschten geronnenen oder nicht-geronnenen Zustand zur Verfügung stehen.

Für die Erreichung des gewünschten Gerinnungszustandes ist es wichtig, ein vorbestimmtes Mengenverhältnis zwischen Flüssigkeit und Präparation einzuhalten.

Bei den im oben genannten Stand der Technik vorgeschlagenen Ausführungsformen befindet sich nach den Probenahmen noch eine relativ große Restmenge an Flüssigkeit in dem Hohlraum der Verschlusskappe.

Durch die typische Aufbereitung einer Probe in einer Zentrifuge wird eine Zugkraft in axialer Richtung auf das verbaute Rückschlagventil ausgeübt und dieses wird dadurch geöffnet. Die im Hohlraum der Verschlusskappe enthaltene relativ große Menge an Flüssigkeit gelangt in das Probenröhrchen und verunreinigt dort das sensible Probengut mit dem bis dahin geronnenen und hämolytischen Blut. Dadurch können die Ergebnisse der späteren Analyse negativ beeinflusst werden.

Auch durch Transportvorgänge, wie sie z. B. in einer Rohrpostanlage auftreten können, besteht die Gefahr der Kontamination der Flüssigkeit durch vor dem Ventil gelagerte Flüssigkeitsreste, die noch von der Blutabnahme stammen.

Die internationale Patentanmeldung WO 2008/066215 offenbart alle Merkmale des Oberbegriffs des Patentanspruchs 1.

### Aufgabe der Erfindung

Der Erfindung liegt die Aufgabe zugrunde, eine bekannte Verschlusskappe zum Verschließen eines Probenröhrchens, ein bekanntes Blutentnahmeröhrchen sowie ein bekanntes Verfahren zur Handhabung des Blutentnahmeröhrchen dahingehend weiterzubilden, dass das Volumen der Flüssigkeit, welches bei einer Zentrifugation des Blutentnahmeröhrchens aus dessen Verschlusskappe in dessen Probenröhrchen abfließt, derart reduziert und begrenzt wird, dass es bei einer späteren Analyse der Flüssigkeit, insbesondere von Blut, zu keiner Störung führt beziehungsweise dass das Ergebnis der Analyse nicht verfälscht wird.

### Erfindung

Diese Aufgabe wird durch den Gegenstand+des Patentanspruchs 1 gelöst. Dieser ist dadurch gekennzeichnet, dass die Trennwand an ihrem der Membrane zugewandten Ende eine Überlaufkante und/oder eine Perforation aufweist zum Ermöglichen der fluidleitenden Kommunikation zwischen dem ersten und dem zweiten Teilraum und dass sich die Trennwand vom Boden des Hohlraums aus in den Hohlraum hinein erstreckt.

Der Hohlraum in der Verschlusskappe ist erfindungsgemäß in mindestens zwei Teilräume unterteilt. Deshalb ist das Volumen jedes einzelnen der Teilräume kleiner als Volumen des ursprünglich gesamten Hohlraums. Erfindungsgemäß kann nur die Flüssigkeitsmenge aus dem zweiten Teilraum bei einer Zentrifugation des Blutentnahmeröhrchens in das Probenröhrchen abfließen, weil nur der zweite Teilraum oberhalb der Öffnung ausgebildet ist. Auf diese Weise wird erfindungsgemäß sichergestellt, dass nur ein Teil des im gesamten Hohlraum der Verschlusskappe befindlichen Flüssigkeitsvolumens in das Probenröhrchen abfließen kann. Insofern ist das in das Probenröhrchen abfließende Flüssigkeitsvolumen gegenüber dem gesamten in dem Hohlraum der Verschlusskappe eingeschlossenen Flüssigkeitsvolumen vorteilhafterweise reduziert.

### Definitionen

- Flüssigkeit:: Flüssigkeiten, die einem menschlichen oder tierischen Körper entnommen werden können, vorzugsweise Blut
- Präparation:: Definierte Menge eines Wirkstoffs (Gerinnungshemmer oder Gerinnungsbeschleuniger) zur Einstellung einer gewünschten Gerinnungseigenschaft einer Flüssigkeit beispielsweise in einem Probenröhrchen
- Rückschlagventil:: kann im Rahmen der vorliegenden Erfindung auch als einstückiges Verschlusselement ausgebildet sein

Das Volumen des zweiten Teilraums und dementsprechend auch die erfindungsgemäß nur noch in das Probenröhrchen abfließende Restmenge an Körperflüssigkeit bzw. Blut sind so klein bemessen, dass die durch sie bedingte nachteilige Beeinflussung des Mengenverhältnisses von Körperflüssigkeit zu Präparation in dem Probenröhrchen vernachlässigbar klein ist; insbesondere ist die Restmenge so klein, dass die spätere Analyse der entnommenen (Blut-) Probe ohne eine nennenswerte Verfälschung möglich ist.

Gemäß einem ersten Ausführungsbeispiel ist das Volumen des ersten Hohlraums größer als das Volumen des zweiten Hohlraums. Der zweite Teilraum ist demnach kleiner als der erste Teilraum. Der kleinere und größere Teilraum bilden zusammen zumindest ein Teilvolumen des gesamten Hohlraums aus. Der Begriff größerer Teilraum schließt nicht aus, dass der größere Teilraum seinerseits nochmals in eine Mehrzahl von kleineren Kammern unterteilt ist, deren einzelne Volumina auch noch kleiner sein können als das Volumen des kleineren Teilraums. Keine der Kammern hat dann aber eine Öffnung in ihrem jeweiligen Boden.

Die oben genannte Aufgabe wird weiterhin gelöst durch das Blutentnahmeröhrchen nach Anspruch 3. Dieses ist dadurch gekennzeichnet, dass es einerseits das Probenröhrchen mit einem verschließbaren Ende zur Aufnahme von der Flüssigkeit und andererseits die Verschlusskappe zum Verschließen des Probenröhrchens an seinem verschließbaren Ende aufweist. Die Verschlusskappe ist ausgebildet nach einem der Ansprüche 1 oder 2.

Die oben genannte Aufgabe wird weiterhin durch das Verfahren zur Handhabung des Blutentnahmeröhrchens nach Anspruch 4 gelöst. Der Vorteil dieses Verfahrens entspricht den oben mit Bezug auf die beanspruchte Verschlusskappe genannten Vorteilen.

Der Beschreibung sind die folgenden sechs Figuren beigefügt:
- Fig. 1: Darstellung des Blutentnahmeröhrchens;
- Fig. 2: Darstellung der durchströmten Verschlusskappe;
- Fig. 3: Darstellung eines Details aus Fig. 2 mit einer Überlaufkante bzw. Perforation;
- Fig. 4 a - d: Darstellung Füllvorgang der Verschlusskappe im zeitlichen Verlauf;
- Fig. 5: Darstellung der Verschlusskappe mit verbundenem Probenröhrchen; und
- Fig. 6: Darstellung eines Querschnitts durch die Verschlusskappe

Die Erfindung wird nachfolgend unter Bezugnahme auf die genannten Figuren in Form von Ausführungsbeispielen detailliert beschrieben. In allen Figuren sind gleiche technische Elemente mit gleichen Bezugszeichen bezeichnet.

### Figurenbeschreibung

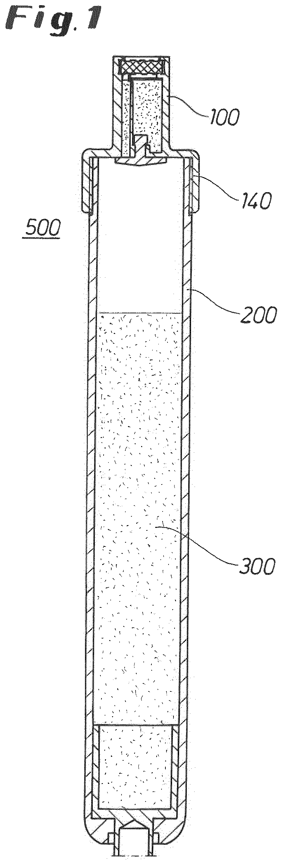

Figur 1 stellt ein Blutentnahmeröhrchen 500 bestehend aus einer Verschlusskappe 100 und einem Probenröhrchen 200 zur Aufnahme einer Flüssigkeit 300 aus einem menschlichen oder tierischen Körper dar.

Die Verschlusskappe 100 weist vorzugsweise ein Verbindungsteil 140 zum Verbinden der Verschlusskappe 100 mit dem Probenröhrchen 200 bzw. zum Verschließen des Probenröhrchens 200 auf. Das Verbindungsteil 140 kann als Schraub-, Bajonett oder Steckverbindung ausgestaltet sein. Die Verbindung muss entsprechend den Anforderungen flüssigkeits- und / oder luftdicht ausgeführt sein. Dazu kann es ggf. notwendig sein, zusätzliche Dichtmittel zu verwenden. Das Verbindungsteil ist eventuell auch ganz entbehrlich, z.B. wenn die Verschlußkappe einfach nur auf das Probenröhrchen aufgesteckt wird.

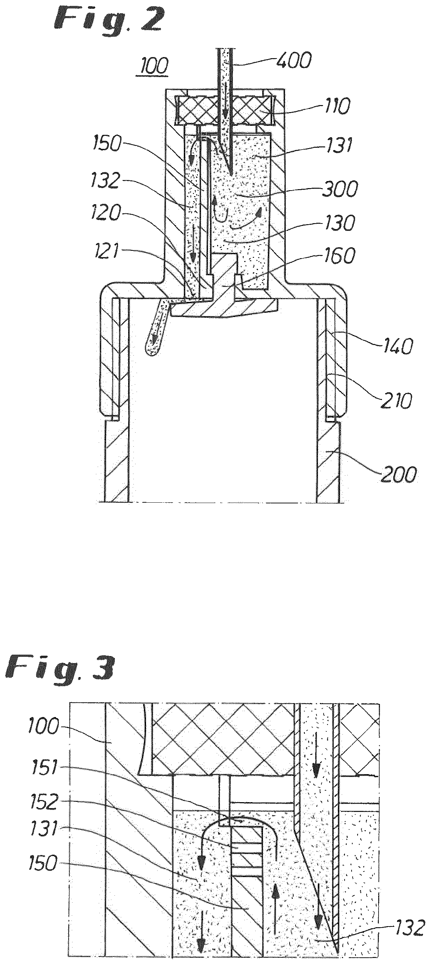

Figur 2 zeigt eine Darstellung der von einer Flüssigkeit 300 durchströmten Verschlusskappe 100, die das Probenröhrchen 200 verschließt. In der Verschlusskappe 100 ist ein Hohlraum 130 ausgebildet, der begrenzt wird von einer Membrane 110 und einem Boden 120. In dem Boden ist eine Öffnung 121 ausgebildet, welche von einem Rückschlagventil 160 geöffnet oder verschlossen werden kann. Das Rückschlagventil 160 ist so gestaltet, dass es die Flüssigkeit 300 aus dem Hohlraum 130 in der Verschlusskappe 100 in das Probenröhrchen 200 fließen lässt, aber die Gegenrichtung sperrt.

Die Membrane 110 ist von einer Kanüle 400 durchstechbar. Die Kanüle 400 stellt eine fluidleitende Verbindung zwischen der Verschlusskappe 100 und dem Körper her, aus dem die Flüssigkeit entnommen wird und leitet die Flüssigkeit 300 in den Hohlraum 130 der Verschlusskappe 100. Der Hohlraum 130 wird durch eine Trennwand 150 in einen ersten, vorzugsweise größeren Teilraum 131 und einen zweiten, vorzugsweise kleineren Teilraum 132 unterteilt. Der größere Teilraum 131 weist keine Öffnung 121 in seinem Boden 120 auf. Der kleinere Teilraum 132 ist über der Öffnung 121 im Boden 120 ausgebildet. Das Volumen des kleineren Teilraums liegt bespielsweise in einem Bereich zwischen 0-70µl (Mikroliter).

Die Ausführung der Trennwand 150 ist so, dass eine durchströmbare Fläche zwischen den beiden Teilräumen 131, 132 entsteht. Die durchströmbare Fläche kann resultieren aus einem Spalt zwischen einer Überlaufkante 151 der Trennwand 150 und der darüber befindlichen Membrane 110 und / oder einer Perforation 152 in der Trennwand 150 (Figur 3). Die durchströmbare Fläche muss so groß sein, dass ein ausreichender Flüssigkeitsstrom bei der Probenentnahme gewährleistet ist. Beide Teilräume 131/132 sind dadurch fluidleitend kommunizierend miteinander verbunden. Die Trennwand 150 erstreckt sich vorzugsweise ausgehend von dem Boden 120 der Verschlusskappe in Richtung der Membran 110.

Die Vektorpfeile in Fig. 2 deuten eine Fließrichtung der Flüssigkeit 300 durch die beiden Teilräume 131/132 und das geöffnete Verschlusselement 161 in das Probenröhrchen 200 an.

Das erfindungsgemäße Verfahren zur Handhabung wird nachfolgend anhand der Figuren 2 bis 5 beschrieben. Mit der Verschlusskappe 100 wird ein verschließbares Ende 210 des Probenröhrchens 200 verschlossen. Vor dem Verschließen kann eine Präparation in das Probenröhrchen 200 gegeben werden. Das optionale Eingeben der Präparation und / oder das notwendige Verschließen des Blutentnahmeröhrchens können herstellerseitig erfolgen. Das so zusammengesetzte Blutentnahmeröhrchen 500 kann mit der Flüssigkeit 300 befüllt werden.

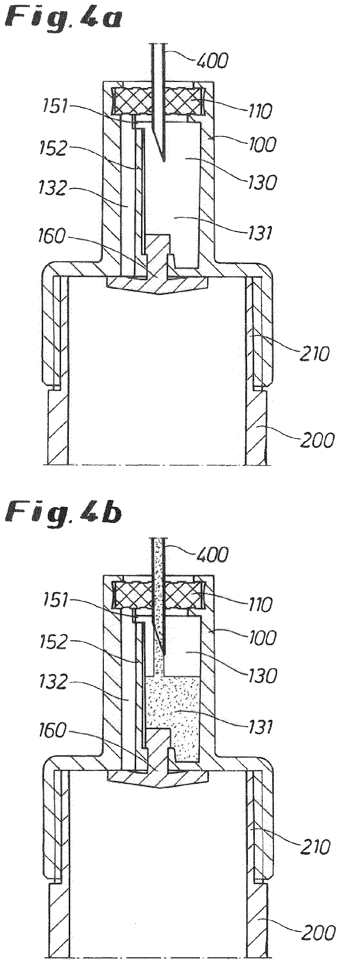

Dazu wird der Körper, z.B. mit einer Kanüle 400, punktiert. Die eine Seite der Kanüle 400 sticht in den Körper, die andere Seite durchsticht die Membrane 110 in der Verschlusskappe 100, siehe Figur 4a.

Die Flüssigkeit 300 strömt aufgrund eines bestehenden Unterdrucks in dem Probenröhrchen aus dem Körper durch die Kanüle 400 in den Hohlraum 130 der Verschlusskappe 100. Je nach Bauart des Blutentnahmeröhrchens wird der Unterdruck darin bereits herstellerseitig dort eingebracht. Weitere Blutentnahmetechniken, wie z.B. das Vakuum-Prinzip oder das Aspirations-Prinzip, sind denkbar. Hierbei ist dann zum Druckaufbau in dem Probenröhrchen eine Interaktion durch den Anwender nötig.

Wenn ein Stempel bzw. Kolben des Probenröhrchens vor der Punktion einer Vene in dem Körper manuell in eine hintere Rastposition gezogen wird, wird dadurch das Vakuum in dem Blutentnahmeröhrchen erst bei Bedarf kurz vor der Blutentnahme aufgebaut und die Abnahme des Blutes erfolgt danach analog zu dem herstellerseitig vorevakuierten Röhrchen mit Vakuumtechnik (Vakuum-Prinzip). Von Aspirationstechnik sprechen wir, wenn zunächst die Kopplung mit der Vene erfolgt und die Blutentnahme parallel zum Kolbenhub geschieht.

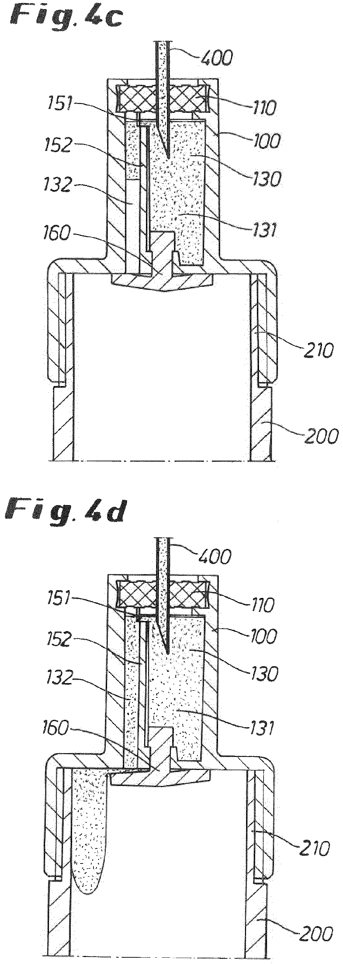

In dem Hohlraum 130 der Verschlusskappe 100 wird vorzugsweise zunächst der große Teilraum 131 befüllt, siehe Figur 4b. Anschließend strömt die Flüssigkeit 300 über die Überlaufkante 151 und / oder durch die Perforation 152 in den kleinen Teilraum 132, siehe Figur 4c. Optional kann auch nur der kleinere Teilraum befüllt werden.

Aufgrund eines bestehenden Druckunterschiedes zwischen dem Unterdruck im Probenröhrchen 200 und dem Druck in der Verschlusskappe 100 öffnet sich das Rückschlagventil 160 bei einer Blutentnahme. Die Flüssigkeit 300 läuft dann aus der Verschlusskappe 100 und insbesondere aus deren kleinerem Teilraum in das Probenröhrchen 200 ab, siehe Figur 4d.

Ist das Probenröhrchen 200 ausreichend mit der Flüssigkeit 300 befüllt, wird die Probenahme unterbrochen, indem die Flüssigkeitsleitung zwischen dem Körper und dem Blutentnahmeröhrchen unterbrochen wird. Dadurch kommt es zu einem Druckausgleich in dem Blutentnahmeröhrchen; insbesondere wird dadurch der Unterdruck im Probenröhrchen abgebaut. Ist der Druckunterschied zwischen dem Blutentnahmeröhrchen 500 und dem Volumen 130 in der Verschlusskappe 100 ausgeglichen, schließt sich dadurch das Rückschlagventil 160 wieder. Das Probenröhrchen 200 ist dann gegenüber der Verschlusskappe fluidleitend verschlossen.

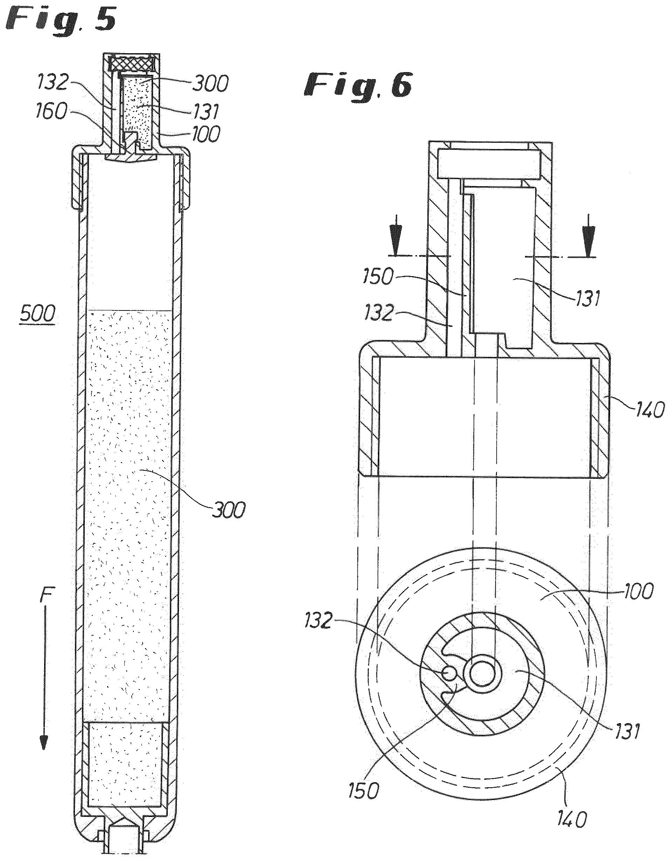

Bei einer, sich an die Probenahme anschließenden, Aufbereitung der Flüssigkeit 300 in dem Blutentnahmeröhrchen 500 in einer Zentrifuge wirkt auf das Blutentnahmeröhrchen 500 eine auf den Boden des Proberöhrchens gerichtete Kraft F; siehe den Pfeil in Fig. 5. Dadurch trennen sich die schweren von den leichten Bestandteilen in der Flüssigkeit 300. Durch diese Kraft F wird, bei einer ausreichenden Größe der Kraft F, auch das Rückschlagventil 160 wieder geöffnet und die Flüssigkeit 300 fließt ungewollt aus dem Hohlraum der Verschlusskappe in das Probenröhrchen 200.

Wie in Figur 5 dargestellt, fließt aber erfindungsgemäß nur das Flüssigkeitsvolumen aus dem zweiten, vorzugsweise kleineren Teilraum 132 in das Probenröhrchen 200 ab. Dagegen verbleibt die Flüssigkeit 300 in dem ersten, vorzugsweise größeren Teilraum 131 in der Verschlusskappe 100 zurück.

Bei einer ausreichend kleinen Dimensionierung des zweiten, vorzugsweise kleineren Teilraums 132 in der Verschlusskappe 100 bezogen auf die Gesamtmenge an Flüssigkeit im Probenröhrchen kann die für die spätere Analyse benötigte Qualität der Flüssigkeit gewährleistet werden, auch wenn die Flüssigkeit 300 aus dem kleineren Teilraum 132 in das Probenröhrchen 200 fließt.

Figur 6 zeigt einen Querschnitt durch die erfindungsgemäße Verschlusskappe 100. Zu erkennen ist insbesondere eine beispielhafte Anordnung und Ausbildung für die Trennwand 150 sowie für den größeren Teilraum 131 und den kleineren Teilraum 132.

### Bezugszeichen

- 100: Verschlusskappe
- 110: Membrane
- 120: Boden
- 121: Öffnung
- 130: Hohlraum
- 131: erster, vorzugsweise größerer Teilraum
- 132: zweiter, vorzugsweise kleinerer Teilraum
- 140: Verbindungsteil
- 150: Trennwand
- 151: Überlaufkante
- 152: Perforation
- 160: Rückschlagventil
- 170: Verschlussbereich
- 200: Probenröhrchen
- 210: verschließbares Ende
- 300: Flüssigkeit
- 400: Kanüle
- 500: Blutentnahmeröhrchen

## Patentansprüche

1. Verschlusskappe (100) zum Verschließen eines Probenröhrchens (200) zur Aufnahme einer Flüssigkeit (300), aufweisend:
- eine von einer Kanüle (400) durchstechbare Membrane (110),
- einen unterhalb der Membrane angeordneten Boden (120) mit einer Öffnung (121), wobei zwischen der Membrane (110) und dem Boden (120) ein Hohlraum (130) ausgebildet ist zur Aufnahme der Flüssigkeit (300); und
- ein Rückschlagventil (160) zum Freigeben der Öffnung (121) in dem Boden (120) zum Abfließen der Flüssigkeit (300) aus dem Hohlraum (130) in das Probenröhrchen (200) hinein und zum Verschließen der Öffnung (121) für eine Fließrichtung der Flüssigkeit (300) aus dem Probenröhrchen (200) in den Hohlraum (130) hinein;
wobei der Hohlraum (130) eine Trennwand (150) aufweist zum Aufteilen des Hohlraums in einen ersten (131) und einen zweiten (132) Teilraum;
wobei der erste Teilraum (131) über einem Bereich des Bodens (120) ohne die Öffnung (121) ausgebildet ist;
wobei der zweite Teilraum (132) über der Öffnung (121) in dem Boden (120) ausgebildet ist;
wobei der erste Teilraum (131) und der zweite (132) Teilraum oberhalb des Bodens (120) fluidleitend miteinander kommunizierend ausgebildet sind; und wobei beim Zentrifugieren nur Flüssigkeit (300) aus dem zweiten Teilraum (132), in dessen Boden (120) die Öffnung (121) ausgebildet ist, in das Probenröhrchen (200) abfließt;
**dadurch gekennzeichnet,**
**dass** die Trennwand (150) an ihrem der Membrane (110) zugewandten Ende eine Überlaufkante (151) und/oder eine Perforation (152) aufweist zum Ermöglichen der fluidleitenden Kommunikation zwischen dem ersten (131) und dem zweiten Teilraum (132); und
**dass** sich die Trennwand (150) vom Boden (120) des Hohlraums (130) aus in den Hohlraum (130) hinein erstreckt.

2. Verschlusskappe (100) nach Anspruch 1,
**dadurch gekennzeichnet,**
**dass** der erste Teilraum (131) größer ist als der zweite Teilraum (132).

3. Blutentnahmeröhrchen (500) aufweisend:
- ein Probenröhrchen (200) mit einem verschließbaren Ende (210) zur Aufnahme von einer Flüssigkeit (300);
- eine Verschlusskappe (100) zum Verschließen des Probenröhrchens an seinem verschließbaren Ende (210); **dadurch gekennzeichnet,**
**dass** die Verschlusskappe (100) ausgebildet ist nach einem der vorangegangenen Ansprüche 1 und 2.

4. Verfahren zum Handhaben eines Blutentnahmeröhrchens (500) nach Anspruch 3, aufweisend folgende Schritte:
- Verschließen des Probenröhrchens (200) an seinem verschließbaren Ende (210) mit der Verschlusskappe (100) nach einem der Ansprüche 1 bis 2;
- Durchstechen der Membrane (110) in der Verschlusskappe (100) mit einem Ende einer Kanüle (400);
- Entnehmen von Flüssigkeit (300) aus einem Körper mit dem anderen Ende der Kanüle (400), wobei die Flüssigkeit (300) durch die Kanüle (400) und die Membrane (110) in den Hohlraum (130) der Verschlusskappe (100) fließt und anschließend durch die durch das Rückschlagventil (160) in Fließrichtung geöffnete Öffnung (121) in dem Boden (120) der Verschlusskappe (100) in das Probenröhrchen strömt und dieses befüllt;
- Entfernen der Kanüle (400) aus der Membrane (110), woraufhin sich die Membrane (110) selbsttätig wieder verschließt; und
- Zentrifugieren des Blutentnahmeröhrchen (500) zum Auftrennen der Flüssigkeit (300) in schwerere und leichtere Bestandteile, wobei das Rückschlagventil (160) die Öffnung (121) im Boden (120) der Verschlusskappe (100) aufgrund einer Zentrifugalkraft freigibt, so dass die Flüssigkeit (300) aus dem Hohlraum (130) in das Probenröhrchen (200) fließt;
wobei bei der Entnahme der Flüssigkeit (300) zumindest der zweite Teilraum (132), vorzugsweise sowohl der erste Teilraum (131) wie auch der zweite (132) Teilraum des Hohlraums (130), die fluidleitend kommunizierend miteinander verbunden sind, mit Flüssigkeit (300) befüllt werden; und
wobei beim Zentrifugieren nur Flüssigkeit (300) aus dem zweiten Teilraum (132), in dessen Boden (120) die Öffnung (121) ausgebildet ist, in das Probenröhrchen (200) abfließt;
**dadurch gekennzeichnet,**
**dass** die Flüssigkeit bei der Entnahme aus dem Körper vorzugsweise zunächst in den ersten Teilraum (131) der Verschlusskappe (100) fließt und diesen befüllt und sodann über den Überlauf (151) und/oder durch die Perforation (152) in den zweiten Teilraum (132) strömt.

5. Verfahren nach Anspruch 4,
**dadurch gekennzeichnet,**
**dass** das Probenröhrchen (200) vor dem Verschließen mit der Verschlusskappe (100) mit einer Präparation für Blut versehen wurde.

## Claims

1. A sealing cap (100) for sealing a sample tube (200) for receiving a liquid (300), comprising:
- a membrane (110) that can be pierced by a cannula (400),
- a base (120) arranged below the membrane and having an opening (121), wherein a cavity (130) is formed between the membrane (110) and the base (120) for receiving the liquid (300); and
- a non-return valve (160) for unblocking the opening (121) in the base (120) to allow the liquid (300) to flow out from the cavity (130) into the sample tube (200) and for sealing the opening (121) for a flow direction of the liquid (300) from the sample tube (200) into the cavity (130);
wherein the cavity (130) has a separating wall (150) for dividing the cavity into a first (131) and a second (132) sub-area;
wherein the first sub-area (131) is formed above a region of the base (120) without the opening (121);
wherein the second sub-area (132) is formed above the opening (121) in the base (120);
wherein the first sub-area (131) and the second sub-area (132) are formed above the base (120) and are connected to one another in a fluid-conducting communicating manner; and wherein, during centrifugation, only liquid (300) from the second sub-area (132), in the base (120) of which the opening (121) is formed, flows out and into the sample tube (200);
**characterized in**
**that** the separating wall (150), at its end facing the membrane (110), comprises an overflow edge (151) and/or a perforation (152) for enabling the fluid-conducting communication between the first (131) and the second sub-area (132); and
in that the separating wall (150) extends from the base (120) of the cavity (130) into the cavity (130).

2. The sealing cap (100) according to claim 1, **characterized in**
**that** the first sub-area (131) is larger than the second sub-area (132).

3. A blood collection tube (500) comprising:
- a sample tube (200) for receiving a liquid (300) and having a sealable end (210);
- a sealing cap (100) for sealing the sample tube at its sealable end (210);
**characterized in**
**that** the sealing cap (100) is formed according to any one of the preceding claims 1 and 2.

4. A method for handling the blood collection tube (500) according to claim 3, comprising the steps of:
- sealing the sample tube (200) at its sealable end (210) with the sealing cap (100) according to any one of claims 1 to 2;
- piercing the membrane (110) in the sealing cap (100) with one end of a cannula (400);
- withdrawing liquid (300) from a body with the other end of the cannula (400), wherein the liquid (300) flows through the cannula (400) and the membrane (110) into the cavity (130) of the sealing cap (100) and subsequently flows through the opening (121) in the base (120) of the sealing cap (100), which is opened in a flow direction by the non-return valve (160), into the sample tube and fills it;
- removing the cannula (400) from the membrane (110), whereupon the membrane (110) automatically seals again; and
- centrifuging the blood collection tube (500) to separate the liquid (300) into heavier and lighter components, wherein the non-return valve (160) unblocks the opening (121) in the base (120) of the sealing cap (100) due to a centrifugal force, such that the liquid (300) flows out of the cavity (130) into the sample tube (200);
wherein, upon the withdrawal of the liquid (300), at least the second sub-area (132), preferably both the first sub-area (131) and the second sub-area (132) of the cavity (130) which are connected to one another in a fluid-conducting communicating manner, are filled with liquid (300);
wherein during centrifugation only liquid (300) from the second sub-area (132), in the base (120) of which the opening (121) is formed, flows into the sample tube (200) ;
**characterized in**
**that** upon withdrawing the liquid from the body, preferably, the liquid preferably first flows into and fills the first sub-area (131) of the sealing cap (100), and then flows via the overflow (151) and/or through the perforation (152) into the second sub-area (132) .

5. The method according to claim 4,
**characterized in**
**that** the sample tube (200) has been provided with a preparation for blood prior to sealing with the sealing cap (100).

## Revendications

1. Capuchon de fermeture (100) pour fermer un tube d'échantillon (200) destiné à contenir un liquide (300), comprenant :
- une membrane (110) perçable par une canule (400),
- une base (120) disposée au-dessous de la membrane avec une ouverture (121), dans lequel une cavité (130) est formée entre la membrane (110) et la base (120) pour recevoir le liquide (300) ; et
- un clapet anti-retour (160) pour libérer l'ouverture (121) dans la base (120) pour évacuer le liquide (300) de la cavité (130) dans le tube d'échantillon (200) et pour fermer l'ouverture (121) pour une direction d'écoulement du liquide (300) depuis le tube d'échantillon (200) vers la cavité (130) ;
dans lequel la cavité (130) présente une cloison (150) pour diviser la cavité en un premier (131) et un deuxième (132) sous-espaces,
le premier sous-espace (131) sur une région du sol (120) sans l'ouverture (121) est formé ;
dans lequel le deuxième sous-espace (132) est formé au-dessus de l'ouverture (121) dans le sol (120) ;
dans lequel le premier sous-espace (131) et le deuxième (132) sous-espace sont conçus pour communiquer l'un avec l'autre de manière conductrice de fluide au-dessus de la base (120) ; et pendant la centrifugation, seul le liquide (300) s'écoule du deuxième sous-espace (132), au fond (120) duquel l'ouverture (121) est formée, dans le tube d'échantillon (200) ;
**caractérisé en ce que**
la cloison (150) présente un bord de débordement (151) et/ou une perforation (152) à son extrémité tournée vers la membrane (110) pour permettre une communication fluidique entre le premier (131) et le deuxième sous-espace (132); et
la cloison (150) s'étend depuis le fond (120) de la cavité (130) jusque dans la cavité (130).

2. Capuchon de fermeture (100) selon la revendication 1, **caractérisé en ce que**
le premier sous-espace (131) est plus grand que le deuxième sous-espace (132).

3. Tube de prélèvement sanguin (500).
- un tube d'échantillon (200) doté d'une extrémité obturable (210) pour contenir un liquide (300) ;
- un capuchon de fermeture (100) pour fermer le tube d'échantillon au niveau de son extrémité obturable (210) ;
**caractérisé en ce que**
le capuchon de fermeture (100) est conçu selon une des revendications précédentes 1 et 2.

4. Procédé de manipulation d'un tube de prélèvement sanguin (500) selon la revendication 3, comprenant les étapes suivantes consistant à :
- fermer le tube d'échantillon (200) à son extrémité obturable (210) avec le capuchon de fermeture (100) selon une des revendications 1 à 2 ;
- percer la membrane (110) dans le capuchon de fermeture (100) avec une extrémité d'une canule (400) ;
- retirer du liquide (300) d'un corps avec l'autre extrémité de la canule (400), dans lequel le liquide (300) s'écoule à travers la canule (400) et la membrane (110) dans la cavité (130) du capuchon de fermeture (100), puis à travers l'ouverture (121) dans le fond (120) du capuchon de fermeture (100), qui est ouverte dans la direction de l'écoulement par le clapet anti-retour (160), coule dans le tube d'échantillon et le remplit;
- retirer la canule (400) de la membrane (110), après quoi la membrane (110) se referme automatiquement ; et
- centrifuger le tube de prélèvement sanguin (500) pour séparer le liquide (300) en composants plus lourds et plus légers, dans lequel le clapet anti-retour (160) libère l'ouverture (121) dans le fond (120) du bouchon de fermeture (100) sous l'effet d'une force centrifuge, de sorte que le liquide (300) s'écoule de la cavité (130) à l'intérieur du tube à échantillon (200);
dans lequel, lorsque le liquide (300) est retiré, au moins le deuxième sous-espace (132), de préférence à la fois le premier sous-espace (131) et le deuxième (132) sous-espace de la cavité (130), qui sont reliés l'un à l'autre de manière de manière conductrice de fluide, sont remplis de liquide (300) à remplir ; et
dans lequel pendant la centrifugation, seul le liquide (300) s'écoule du deuxième sous-espace (132), au fond (120) duquel l'ouverture (121) est formée, dans le tube d'échantillon (200),
**caractérisé en ce que**
le liquide est éliminé du corps s'écoule de préférence d'abord dans le premier sous-espace (131) du bouchon de fermeture (100) et le remplit puis s'écoule dans le deuxième sous-espace (132) via le trop-plein (151) et/ou à travers la perforation (152).

5. Procédé selon la revendication 4, **caractérisé en ce que** le tube de prélèvement (200) a été muni d'une préparation pour sang avant d'être fermé avec le capuchon de fermeture (100).
